# FASCICULE DE BREVET EUROPEEN

(11) **EP 3 273 859 B1**
(45) Date de publication et mention de la délivrance du brevet: **26.12.2018**
(21) Numéro de dépôt: 16719515.5
(22) Date de dépôt: 25.03.2016
(51) Int. Cl.: B29C 45/14, B29L 31/00, A61B 5/15, A61B 5/153, A61B 5/154

(54) **CONNECTEUR DE SECURITE A AIGUILLE**
SICHERHEITSVERBINDER MIT NADEL
SAFETY CONNECTOR WITH NEEDLE

(30) Priorité: 25.03.2015 FR 1552473
(43) Date de publication de la demande: 31.01.2018
(73) Titulaire: P2A Medical, 74500 Maxilly Sur Leman (FR)
(72) Inventeur: ORLU, Alain, F-74540 Viuz La Chiesaz (FR)
(74) Mandataire: Cabinet Poncet
(86) Numéro de dépôt international: PCT/IB2016/051707
(87) Numéro de publication internationale: WO 2016/151540

(56) Documents cités:
- EP-A1- 1 557 124
- EP-A1- 2 070 476
- JP-A- H11 290 298

## Description

### DOMAINE TECHNIQUE DE L'INVENTION

La présente invention concerne les connecteurs de sécurité permettant de connecter un récipient de prélèvement à une canalisation de conduction de fluide. L'invention concerne en particulier un tel connecteur destiné au prélèvement sanguin dans les applications du domaine de la santé.

Lors de prélèvements sanguins, notamment pour les opérations de dons de sang, on doit systématiquement effectuer une analyse de sang en prélevant une petite quantité de sang dans un récipient de prélèvement. Pour cela, une canalisation de conduction de fluide doit être raccordée de manière amovible et étanche à un récipient de prélèvement, dans des conditions d'hygiène absolue, en nécessitant un minimum de mouvement de l'opérateur pour la connexion du récipient de prélèvement à la canalisation et pour sa déconnexion.

Simultanément, il faut assurer la protection de l'opérateur contre tout risque de blessure et de contamination par le sang prélevé.

Pour cela, on a déjà imaginé un connecteur de sécurité à aiguille, tel que décrit dans le document EP 1 557 124, comprenant une aiguille creuse de passage de fluide, un manchon support d'aiguille, et un manchon protecteur tubulaire s'étendant selon un axe longitudinal, le manchon support d'aiguille portant l'aiguille en la raccordant de manière étanche à une canalisation de conduction de fluide à laquelle il est fixé, une portion dépassante d'aiguille prolongeant axialement le manchon support d'aiguille à l'opposé de la canalisation de conduction de fluide, le manchon protecteur tubulaire ayant un espace intérieur contenant la portion dépassante d'aiguille, avec une extrémité distale ouverte pour l'introduction d'un récipient de prélèvement à connecter par l'aiguille, et avec une extrémité proximale que traverse le manchon support d'aiguille. Le manchon protecteur tubulaire comporte un tronçon proximal se développant depuis son extrémité proximale fermée en correspondance de la portion dépassante d'aiguille, et comporte un tronçon distal prolongeant le tronçon proximal entre la portion dépassante d'aiguille et l'extrémité distale du manchon protecteur tubulaire.

L'aiguille est ainsi contenue dans le manchon protecteur tubulaire, à distance de l'extrémité distale ouverte, ce qui limite le risque pour l'opérateur de se piquer avec celle-ci. Pour encore améliorer la sécurité de l'opérateur, le connecteur du document EP 1 557 124 comporte un clapet rabattable permettant d'obturer l'extrémité distale ouverte du manchon protecteur tubulaire. En position d'obturation, le clapet rabattable empêche l'utilisateur d'introduire un doigt dans le manchon protecteur tubulaire en direction de l'extrémité acérée de l'aiguille.

L'inconvénient du connecteur du document EP 1 557 124 est que la fabrication du clapet rabattable augmente significativement son coût de fabrication, ne serait-ce que par la quantité de matière supplémentaire destinée à constituer le clapet rabattable.

Le clapet rabattable peut en outre accidentellement s'ouvrir lors de la manipulation ou du transport du connecteur, de sorte qu'il peut être ouvert lorsque l'opérateur effectue la préhension du connecteur. Il y a alors un risque que l'opérateur, lors de la préhension, engage par mégarde l'un de ses doigts dans le manchon protecteur et se pique sur l'aiguille.

Le document antérieur JP H3-111307 U, qui est par ailleurs cité par le document antérieur JP H11-290298 A, décrit un connecteur de sécurité à aiguille dont la canalisation de conduction de fluide se présente sous la forme d'une aiguille destinée à pénétrer dans une veine du patient. Cette aiguille est en fait une aiguille double dont l'autre extrémité dépasse dans l'espace intérieur d'un manchon protecteur tubulaire à extrémité distale ouverte pour l'introduction d'un récipient de prélèvement à connecter par l'aiguille. Une gaine de protection entoure la partie d'aiguille dépassant dans le manchon protecteur tubulaire. Le retroussement de cette gaine peut mener à une compression élastique de celle-ci, qui peut parfois avoir tendance à reprendre sa forme en éjectant le récipient de prélèvement. Pour éviter une telle éjection, le document JP H3-111307 U décrit une bague de verrouillage rapportée oblongue déformable élastiquement et engagée dans deux ouvertures latérales diamétralement opposées du manchon protecteur tubulaire. Cette bague n'est pas conçue pour s'opposer à la pénétration du doigt d'un utilisateur, car elle est prévue au contraire pour se déformer facilement afin de laisser passer un récipient de prélèvement introduit dans le manchon protecteur tubulaire. Cette bague est fabriquée séparément du manchon protecteur tubulaire, ce qui complexifie et rend plus onéreux le procédé de fabrication. Et cette bague de verrouillage peut également être accidentellement perdue. D'ailleurs, probablement en vue de limiter le risque de perte accidentelle, il est prévu un jeu fonctionnel de sorte que la bague de verrouillage dépasse en permanence au moins en partie dans l'espace intérieur, même lors du passage du bouchon de récipient de prélèvement dans la bague élastique lorsqu'un récipient de prélèvement est introduit dans le manchon protecteur tubulaire.

### EXPOSE DE L'INVENTION

Un problème proposé par la présente invention est de concevoir un connecteur de sécurité à aiguille permettant de limiter efficacement les risques de piqûre accidentelle pour l'opérateur.

Simultanément, l'invention vise à assurer une protection efficace de l'opérateur, avant et après un prélèvement, à moindre coût.

Pour atteindre ces objets ainsi que d'autres, l'invention propose un connecteur de sécurité à aiguille, comprenant une aiguille creuse de passage de fluide, un manchon support d'aiguille, et un manchon protecteur tubulaire s'étendant selon un axe longitudinal, l'aiguille étant raccordée de manière étanche à une canalisation de conduction de fluide à laquelle est fixé le manchon support d'aiguille, une portion dépassante d'aiguille prolongeant axialement le manchon support d'aiguille à l'opposé de la canalisation de conduction de fluide, le manchon protecteur tubulaire ayant un espace intérieur défini par une paroi périphérique de manchon et contenant la portion dépassante d'aiguille, avec une extrémité distale ouverte pour l'introduction d'un récipient de prélèvement à connecter par l'aiguille, et avec une extrémité proximale que traverse le manchon support d'aiguille, le manchon protecteur tubulaire comportant un tronçon proximal à paroi périphérique proximale et se développant depuis son extrémité proximale en correspondance de la portion dépassante d'aiguille, et comportant un tronçon distal à paroi périphérique distale et prolongeant le tronçon proximal entre la portion dépassante d'aiguille et l'extrémité distale du manchon protecteur tubulaire ;
selon l'invention :
- la paroi périphérique distale comporte au moins une portion de paroi sélectivement déplaçable entre une position de retrait et une position de protection,
- en position de retrait, la portion de paroi déplaçable ne dépasse pas dans l'espace intérieur,
- en position de protection, la portion de paroi déplaçable fait saillie dans l'espace intérieur pour constituer un obstacle obstruant au moins en partie l'espace intérieur dans le tronçon distal.

La portion de paroi déplaçable étant ménagée dans la paroi latérale périphérique du tronçon distal, sa fabrication ne nécessite pas d'ajout de matière par rapport à ce qui est nécessaire pour la fabrication du manchon protecteur tubulaire. L'obstacle, que peut former la portion de paroi déplaçable pour obstruer au moins en partie l'espace intérieur dans le tronçon distal, est formé d'une seule pièce avec la paroi périphérique distale. Cet obstacle est ainsi fabriqué de façon économique, sans nécessiter d'ajout de matière par rapport à ce qui est nécessaire pour la fabrication du manchon protecteur tubulaire, et ne peut en aucun cas être perdu accidentellement.

Lorsque le connecteur est livré à l'opérateur, la portion de paroi déplaçable peut déjà être disposée en position de saillie, de façon à interdire toute pénétration d'un doigt de l'opérateur dans le manchon protecteur tubulaire jusqu'à l'aiguille.

Avant d'introduire un tube de prélèvement, l'opérateur déplace la portion de paroi déplaçable en position de retrait. La portion de paroi déplaçable ne dépasse plus dans l'espace intérieur du manchon protecteur tubulaire, qui peut alors recevoir un tube de prélèvement.

Après avoir retiré le tube de prélèvement à l'issue du prélèvement, l'opérateur déplace la portion de paroi déplaçable en position de saillie. La portion de paroi déplaçable obture alors à nouveau partiellement l'extrémité distale ouverte du manchon protecteur tubulaire pour éviter que le doigt d'un opérateur puisse pénétrer dans le manchon protecteur tubulaire jusqu'à venir se piquer sur l'aiguille.

Avantageusement, pour mieux réduire le risque qu'un opérateur se pique, en position de protection, la portion de paroi déplaçable peut être au moins en partie située au voisinage de l'axe longitudinal, en prolongement de la portion dépassante d'aiguille dans l'espace intérieur du manchon protecteur tubulaire. En position de saillie, la portion de paroi déplaçable est ainsi disposée, selon l'axe longitudinal et perpendiculairement à l'axe longitudinal, au moins en partie entre l'extrémité de l'aiguille et l'extrémité distale ouverte du manchon protecteur. Quel que soit le diamètre du doigt de l'opérateur, celui-ci vient ainsi obligatoirement en contact avec la portion de paroi déplaçable qui l'empêche de venir se piquer sur l'aiguille.

Dans un mode de réalisation particulier de l'invention, on peut prévoir que :
- la portion de paroi déplaçable comporte des premier et deuxième volets ménagés dans la paroi périphérique distale,
- les premier et deuxième volets sont respectivement articulés au reste de la paroi périphérique distale selon une première et une seconde zones de charnière respectives autorisant un pivotement des premier et deuxième volets autour d'un premier et d'un deuxième axes de pivotement respectifs parallèles à l'axe longitudinal,
- les premier et deuxième volets sont articulés l'un à l'autre selon une troisième zone de charnière autorisant un pivotement relatif des premier et deuxième volets l'un par rapport à l'autre autour d'un troisième axe de pivotement parallèle à l'axe longitudinal,

- les premier et deuxième volets sont déplaçables à pivotement, autour des premier et deuxième axes de pivotement, entre une position de retrait et une position de protection,
- en position de retrait, les premier et deuxième volets sont alignés avec la paroi périphérique du tronçon proximal de manchon protecteur tubulaire,
- en position de protection, les premier et deuxième volets font saillie dans l'espace intérieur du manchon protecteur tubulaire.

De tels volets sont faciles à fabriquer et à manipuler par un opérateur.

Du fait de l'orientation des axes de pivotement parallèles à l'axe longitudinal, le déplacement de la portion de paroi déplaçable est nécessairement perpendiculaire à l'axe longitudinal. Il en résulte qu'un effort axial exercé par un doigt d'utilisateur sur la portion de paroi déplaçable ne risque aucunement de provoquer l'escamotage de la portion de paroi déplaçable et la pénétration du doigt jusqu'à l'aiguille.

Avantageusement, les première, deuxième et troisième zones de charnière peuvent être réalisées au moyen d'un amincissement local de l'épaisseur de matériau de la paroi périphérique de manchon. De tels amincissements peuvent être réalisés facilement, sans opération de reprise, lors de l'injection du manchon protecteur tubulaire lorsque celui-ci est fabriqué en matière plastique. De bons résultats ont été obtenus avec un manchon protecteur tubulaire fabriqué en polypropylène ou en polyacétal.

De préférence, on peut prévoir que :
- le manchon protecteur tubulaire a une section transversale circulaire,
- les premier et deuxième axes de pivotement sont séparés l'un de l'autre par un secteur angulaire inférieur à 180°.

On est ainsi certain que, lorsque les premier et deuxième volets sont en position de saillie, ils sont dans une position d'équilibre stable. Cela signifie que, partant de leur position de saillie, les volets doivent d'abord être pivotés selon une certaine amplitude angulaire (par l'application d'une force extérieure) depuis leur position de saillie vers leur position de retrait, avant qu'ils ne puissent se déplacer par eux-mêmes jusqu'en position de retrait (en l'absence d'une force extérieure). Cela réduit le risque que les premier et deuxième volets reviennent accidentellement en position de retrait sous l'effet d'un choc ou de vibrations lors du transport ou des manipulations.

Des résultats satisfaisants de protection ont été obtenus avec des premier et deuxième axes de pivotement séparés l'un de l'autre par un secteur angulaire compris entre environ 120° et environ 170°.

Avantageusement, la portion de paroi déplaçable peut s'étendre, le long de l'axe longitudinal, selon une distance comprise entre 12 mm environ à 15 mm environ.

Une telle portion de paroi déplaçable présente une hauteur suffisante pour être facilement enfoncée par le doigt d'un opérateur dans l'espace intérieur du manchon protecteur tubulaire, depuis sa position de retrait vers sa position de saillie.

De préférence, on peut prévoir que :
- le connecteur de sécurité à aiguille comporte des moyens de poussée, ménagés dans la paroi périphérique distale et/ou dans la paroi périphérique proximale, et situés au moins en partie en correspondance de la portion de paroi déplaçable,
- les moyens de poussée sont au moins en partie déplaçables transversalement entre une position de repos et au moins une position de poussée, en étant rappelés élastiquement vers leur position de repos,
- en position de repos, les moyens de poussée ne dépassent pas dans l'espace intérieur et sont de préférence alignés avec la paroi périphérique proximale,
- en position de poussée, les moyens de poussée font saillie dans l'espace intérieur du manchon protecteur tubulaire pour solliciter la portion de paroi déplaçable et la rappeler en position de retrait.

De tels moyens de poussée permettent à l'opérateur de déplacer la portion de paroi déplaçable depuis sa position de saillie vers sa position de retrait sans avoir à introduire un doigt dans l'extrémité distale ouverte du manchon protecteur tubulaire. Pour déplacer la portion de paroi déplaçable depuis sa position de saillie vers sa position de retrait, il suffit que l'opérateur presse radialement la partie latérale dans laquelle sont ménagés les moyens de poussée. Sous les doigts de l'opérateur se trouve en permanence une épaisseur de matière (égale à l'épaisseur de matière du manchon protecteur tubulaire), de sorte que le risque de piqûre des doigts de l'opérateur lors de la manipulation des moyens de poussée est faible.

Les moyens de poussée étant ménagés dans la paroi latérale périphérique du manchon protecteur tubulaire, leur fabrication ne nécessite pas de matière supplémentaire par rapport à un manchon protecteur tubulaire de l'art antérieur.

De préférence, on peut prévoir que :
- les moyens de poussée se présentent sous la forme d'une languette s'étendant selon l'axe longitudinal entre une première extrémité et une deuxième extrémité libre,
- la languette est articulée à sa première extrémité sur le reste de la paroi périphérique proximale et/ou de la paroi périphérique distale selon une quatrième zone de charnière autorisant un pivotement de la languette autour d'un quatrième axe de pivotement perpendiculaire à l'axe longitudinal,
- la deuxième extrémité libre de la languette est située en correspondance de la portion de paroi déplaçable.

Avantageusement, la deuxième extrémité libre de la languette peut comporter une zone de poussée qui s'étend, le long de l'axe longitudinal, selon une distance comprise entre 12 mm environ et 15 mm environ, et qui s'étend, perpendiculairement à l'axe longitudinal, selon une distance comprise entre 10 mm environ et 18 mm environ.

L'extrémité libre de la languette présente ainsi des dimensions suffisantes pour être facilement enfoncée par le doigt d'un opérateur dans l'espace intérieur du manchon protecteur tubulaire, depuis la position de repos vers la position de poussée.

Pour une bonne efficacité lors de leur utilisation afin de déplacer la portion de paroi déplaçable depuis sa position de saillie vers sa position de retrait, les moyens de poussée peuvent être situés de façon diamétralement opposée à la portion de paroi déplaçable par rapport à l'axe longitudinal.

De préférence, on peut prévoir que :
- le manchon protecteur tubulaire comporte, à son extrémité distale ouverte, un épanouissement radial,
- la portion de paroi déplaçable est ménagée, selon l'axe longitudinal, à proximité immédiate de l'extrémité distale ouverte.

L'épanouissement radial constitue une nervure annulaire de renforcement du manchon protecteur tubulaire. L'extrémité distale ouverte du manchon protecteur tubulaire conserve ainsi mieux la forme de sa section transversale pour qu'un tube de prélèvement puisse y être engagé. Et cela contribue à conserver la portion de paroi déplaçable en une position de saillie stable.

Avantageusement, le manchon protecteur tubulaire peut être surmoulé sur le manchon support d'aiguille. La liaison par surmoulage entre le manchon protecteur tubulaire et le manchon support d'aiguille assure une bonne étanchéité du manchon protecteur tubulaire au voisinage de son extrémité proximale. Cela permet de bien retenir dans le manchon protecteur tubulaire d'éventuelles gouttes de sang.

Selon un autre aspect de l'invention, il est proposé un procédé de fabrication d'un connecteur de sécurité à aiguille tel que décrit précédemment. Selon l'invention, ce procédé de fabrication comporte une étape d'injection de matière plastique au cours de laquelle on forme, en une seule fois, le manchon protecteur tubulaire muni :
- de découpes, et le cas échéant d'amincissements d'épaisseur de matière, formant la portion de paroi déplaçable,
- le cas échéant, de découpes formant les moyens de poussée.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue de côté d'un mode de réalisation particulier d'un connecteur de sécurité à aiguille selon l'invention, avec des premier et deuxième volets en position de retrait ;
- la figure 2 est une vue de côté en coupe longitudinale du connecteur de sécurité à aiguille de la figure 1 ;
- la figure 3 est une vue de côté du connecteur de sécurité à aiguille de la figure 1, prise selon une direction perpendiculaire à la direction de la vue de la figure 1 ;
- la figure 4 est une vue de côté du connecteur de sécurité à aiguille de la figure 1, prise selon une direction perpendiculaire à la direction de la vue de la figure 1 et opposée à la direction de la vue de la figure 3 ;
- la figure 5 est une vue de dessus du connecteur de sécurité à aiguille de la figure 1 ;
- la figure 6 est une vue de côté en coupe longitudinale du connecteur de sécurité à aiguille de la figure 1, avec les premier et deuxième volets en position de saillie ;
- la figure 7 est une vue en perspective du connecteur de sécurité à aiguille de la figure 6 ;
- la figure 8 est vue de dessus du connecteur de sécurité à aiguille de la figure 6 ;
- la figure 9 est une vue de côté en coupe longitudinale du connecteur de sécurité à aiguille de la figure 1, avec des moyens de poussée repoussant les premier et deuxième volets depuis leur position de saillie vers leur position de retrait ;
- la figure 10 est une vue en perspective du connecteur de sécurité à aiguille de la figure 9 ; et
- la figure 11 est une vue de côté en coupe longitudinale d'un autre mode de réalisation particulier d'un connecteur de sécurité à aiguille selon l'invention.

### DESCRIPTION DES MODES DE REALISATION PREFERES

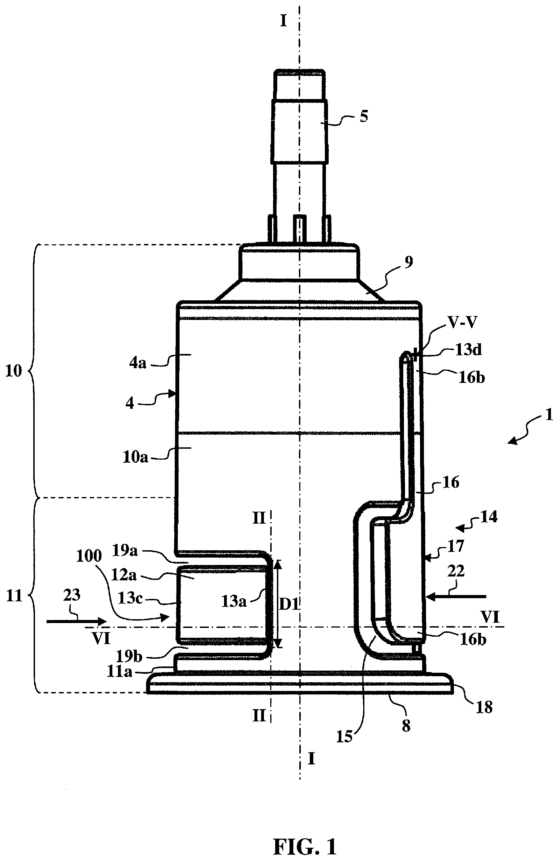

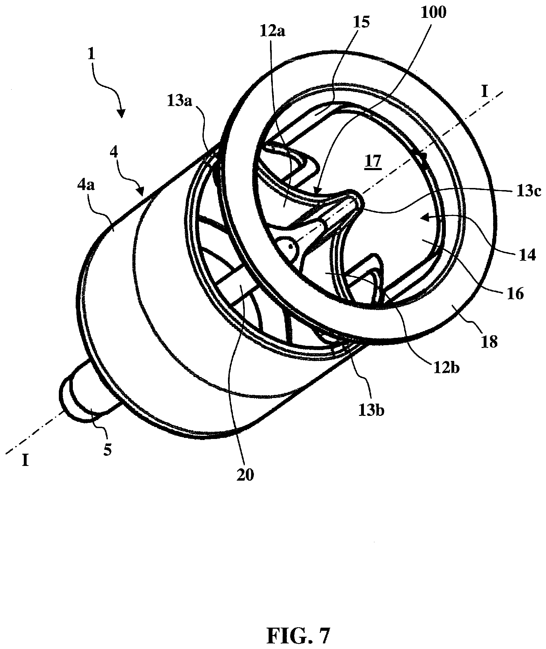

Sur les figures 1 à 7 est illustré un mode de réalisation particulier de connecteur de sécurité 1 à aiguille selon l'invention. Ce connecteur de sécurité 1 à aiguille comprend une aiguille 2 creuse de passage de fluide, un manchon 3 support d'aiguille, et un manchon protecteur 4 tubulaire s'étendant selon un axe longitudinal I-I. Le manchon 3 support d'aiguille porte l'aiguille 2 en la raccordant de manière étanche à une canalisation 5 de conduction de fluide à laquelle il est fixé. En l'espèce, le manchon 3 support d'aiguille est formé d'une seule pièce avec la canalisation 5 de conduction de fluide, l'ensemble formé par le manchon 3 support d'aiguille et la canalisation 5 de conduction de fluide étant surmoulé autour de l'aiguille 2. Le manchon protecteur 4 tubulaire est surmoulé sur le manchon 3 support d'aiguille.

Le connecteur de sécurité 1 comporte une portion dépassante d'aiguille 6 prolongeant axialement le manchon 3 support d'aiguille à l'opposé de la canalisation 5 de conduction de fluide. Le manchon protecteur 4 tubulaire a un espace intérieur 7 défini par une paroi périphérique de manchon 4a et contenant la portion dépassante d'aiguille 6, avec une extrémité distale 8 ouverte pour l'introduction d'un récipient de prélèvement à connecter par l'aiguille 2, et avec une extrémité proximale 9 que traverse le manchon 3 support d'aiguille. Le manchon protecteur 4 tubulaire comporte un tronçon proximal 10 à paroi périphérique proximale 10a et se développant depuis l'extrémité proximale 9 en correspondance de la portion dépassante d'aiguille 6. Le manchon protecteur 4 tubulaire comporte en outre un tronçon distal 11 à paroi périphérique distale 11a et prolongeant le tronçon proximal 10 entre la portion dépassante d'aiguille 6 et l'extrémité distale 8 du manchon protecteur 4 tubulaire. En d'autres termes, la paroi périphérique de manchon 4a est constituée par la réunion des parois périphériques proximale 10a et distale 11a.

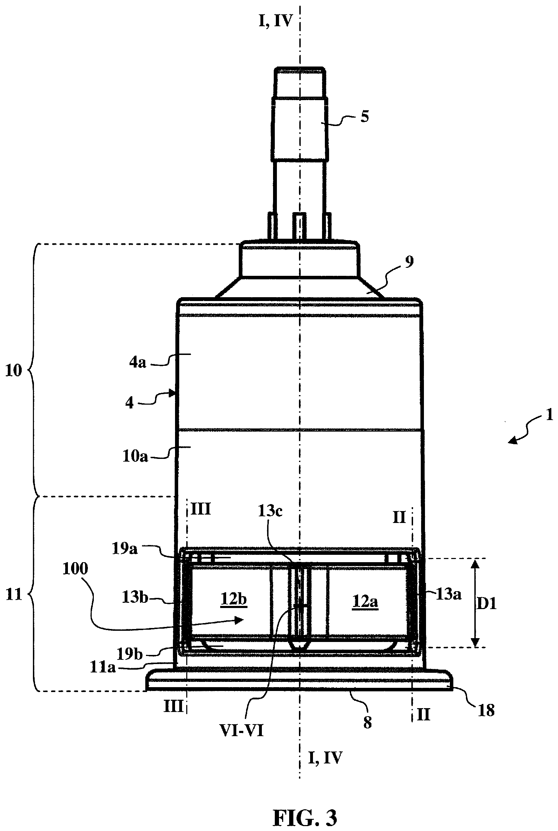

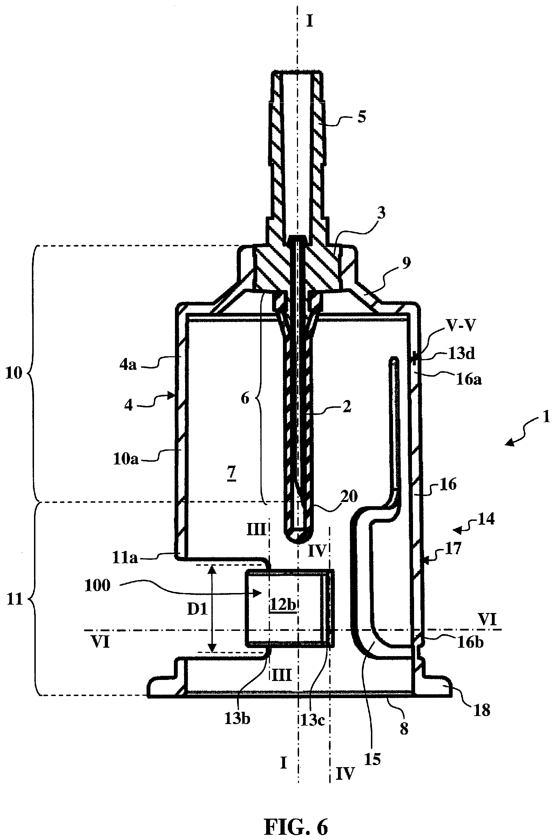

On voit plus particulièrement sur la figure 3 que le manchon protecteur 4 tubulaire comporte des premier et deuxième volets 12a et 12b ménagés dans la paroi latérale périphérique distale 11a. Les volets 12a et 12b forment une portion de paroi déplaçable 100 qui est sélectivement déplaçable entre une position de retrait (figures 1 à 5) et une position de protection (figures 6 à 8).

En position de retrait, la portion de paroi déplaçable 100 ne dépasse pas dans l'espace intérieur 7. En position de protection, la portion de paroi déplaçable 100 fait saillie dans l'espace intérieur 7 pour constituer un obstacle obstruant au moins en partie l'espace intérieur 7 dans le tronçon distal 11.

Le volet 12a est articulé au reste de la paroi périphérique distale 11a selon une première zone de charnière 13a, tandis que le volet 12b est articulé au reste de la paroi périphérique distale 11a selon une seconde zone de charnière 13b. La première zone de charnière 13a autorise un pivotement du premier volet 12a autour d'un premier axe de pivotement II-II parallèle à l'axe longitudinal I-I, tandis que la seconde zone de charnière 13b autorise un pivotement du deuxième volet 12b autour d'un deuxième axe de pivotement III-III parallèle à l'axe longitudinal I-I.

Les premier et deuxième volets 12a et 12b sont également articulés l'un à l'autre selon une troisième zone de charnière 13c autorisant un pivotement relatif des premier et deuxième volets 12a et 12b l'un par rapport à l'autre autour d'un troisième axe de pivotement IV-IV parallèle à l'axe longitudinal I-I. Les premier et deuxième volets 12a et 12b sont ainsi déplaçables à pivotement, autour des premier et deuxième axes de pivotement II-II et III-III, entre une position de retrait (figures 1 à 5) et une position de protection (figures 6 à 8). En position de retrait (figures 1 à 5), les premier et deuxième volets 12a et 12b sont alignés avec la paroi périphérique proximale 10a. En position de protection (figures 6 à 8), les premier et deuxième volets 12a et 12b font saillie dans l'espace intérieur 7 du manchon protecteur 4 tubulaire.

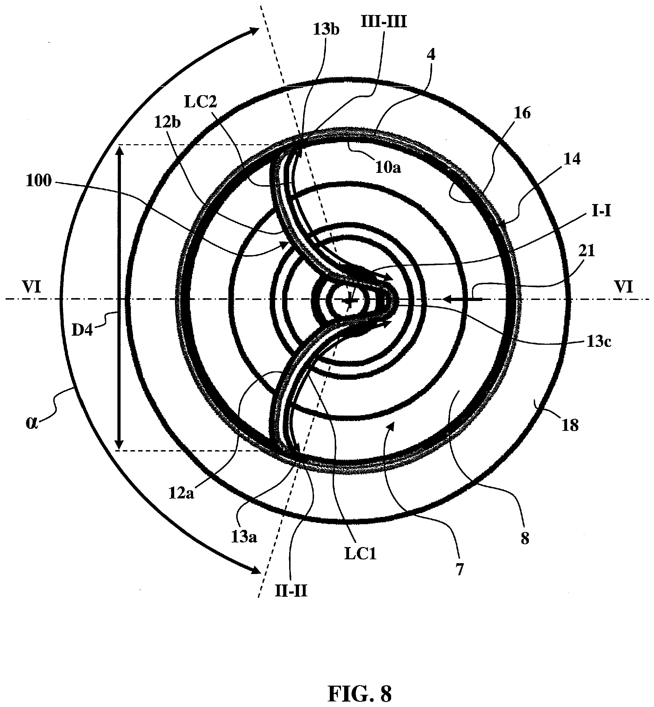

On voit plus particulièrement sur les figures 6 et 8 que, en position de protection, les volets 12a et 12b sont au moins en partie situés au voisinage de l'axe longitudinal I-I, en prolongement de la portion dépassante d'aiguille 6 dans l'espace intérieur 7 du manchon protecteur 4 tubulaire.

Toujours sur la figure 8, on voit que le manchon protecteur 4 tubulaire a une section transversale circulaire. Les premier et deuxième axes de pivotement II-II et III-III sont séparés l'un de l'autre par un secteur angulaire α inférieur à 180°. En l'espèce, le secteur angulaire α est compris entre environ 120° et environ 170°.

Sur la figure 3, on voit que les volets 12a et 12b s'étendent, le long de l'axe longitudinal I-I, selon une distance D1 comprise entre 12 mm environ et 15 mm environ.

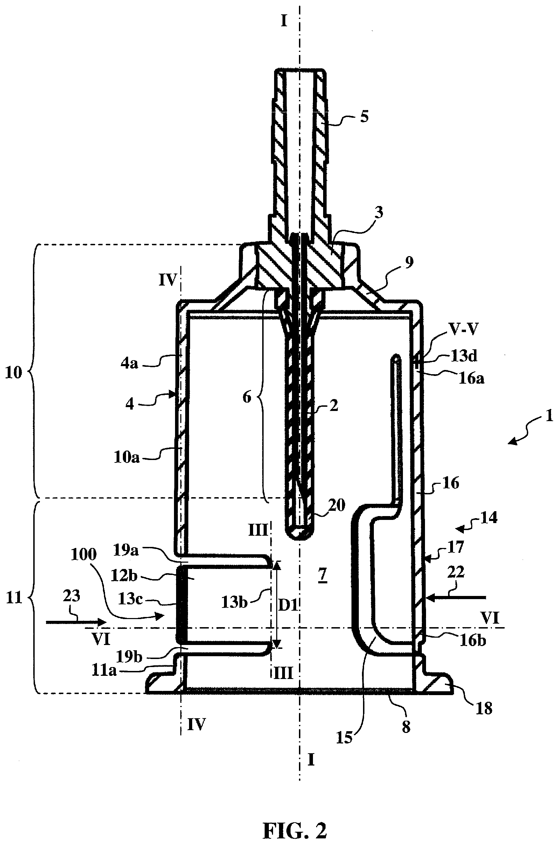

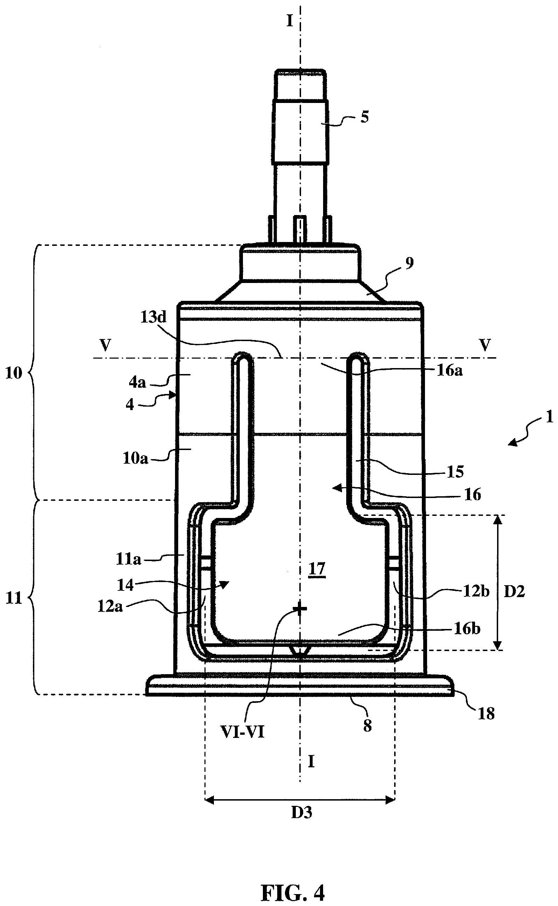

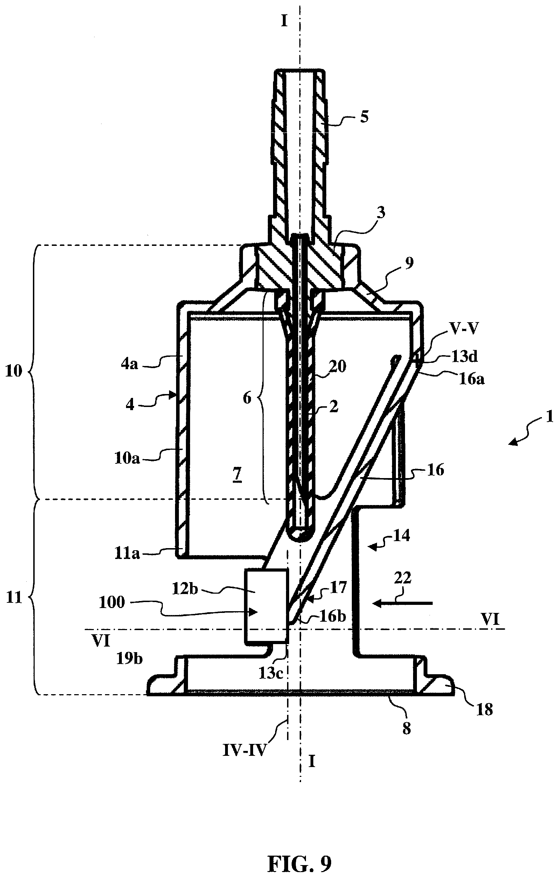

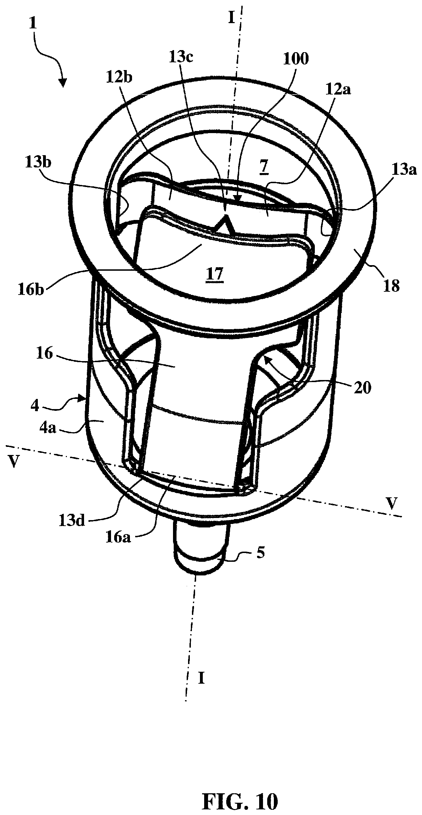

On voit plus particulièrement sur la figure 4 que le connecteur de sécurité 1 à aiguille comporte des moyens de poussée 14, ménagés dans la paroi périphérique distale 11a et dans la paroi périphérique proximale 10a au moyen d'une découpe 15. Les moyens de poussée 14 sont situés en partie en correspondance des volets 12a et 12b selon un axe transversal VI-VI perpendiculaire à l'axe longitudinal I-I. Les moyens de poussée 14 sont au moins en partie déplaçables transversalement, selon l'axe transversal VI-VI, entre une position de repos (figures 2, 4, 5 et 6) et au moins une position de poussée (figures 9 et 10), en étant rappelés élastiquement vers leur position de repos. En position de repos (figures 2, 4, 5 et 6), les moyens de poussée 14 sont alignés avec la paroi périphérique proximale 10a. En position de poussée (figures 9 et 10), les moyens de poussée 14 font saillie dans l'espace intérieur 7 du manchon protecteur 4 tubulaire pour solliciter les volets 12a et 12b et les rappeler en position de retrait.

De façon plus spécifique, les moyens de poussée 14 se présentent sous la forme d'une languette 16 (figure 4) s'étendant selon l'axe longitudinal I-I entre une première extrémité 16a et une deuxième extrémité 16b libre. La languette 16 est articulée à sa première extrémité 16a sur le reste de la paroi périphérique proximale 10a selon une quatrième zone de charnière 13d autorisant un pivotement de la languette 16 autour d'un quatrième axe de pivotement V-V perpendiculaire à l'axe longitudinal I-I. La deuxième extrémité 16b libre de la languette 16 est située en correspondance des volets 12a et 12b.

Dans le mode de réalisation illustré plus particulièrement sur les figures 2 et 4, la deuxième extrémité libre 16b de la languette 16 comporte une zone de poussée 17 qui s'étend, le long de l'axe longitudinal I-I, selon une distance D2 comprise entre 12 mm environ et 15 mm environ. Cette zone de poussée 17 s'étend également, perpendiculairement à l'axe longitudinal I-I, selon une distance D3 comprise entre 10 mm environ et 18 mm environ.

On voit plus particulièrement sur les figures 2 et 10 que les moyens de poussée 14 sont situés de façon diamétralement opposée à la troisième zone de charnière 13c par rapport à l'axe longitudinal I-I.

Les première, deuxième et troisième zones de charnière 13a à 13c sont réalisées au moyen d'un amincissement local de l'épaisseur de matériau de la paroi périphérique 4a du manchon protecteur 4 tubulaire. Le manchon protecteur 4 tubulaire est fabriqué en matière plastique.

On voit plus particulièrement sur les figures 1 à 3 que le manchon protecteur 4 tubulaire comporte, à son extrémité distale 8 ouverte, un épanouissement radial 18. Les premier et deuxième volets 12a et 12b sont ménagés, selon l'axe longitudinal I-I, à proximité immédiate de l'extrémité distale 8 ouverte. On entend par « proximité immédiate » une distance comprise entre 1 mm et 5 mm.

Pour fabriquer le connecteur 1 de sécurité à aiguille tel qu'illustré sur les figures 1 à 10, il est possible de procéder à une étape unique d'injection de matière plastique au cours de laquelle on forme, en une seule fois, le manchon protecteur 4 tubulaire muni :
- de découpes 19a et 19b formant les volets 12a et 12b,
- d'amincissements d'épaisseur de matière formant les première, deuxième et troisième zones de charnières 13a à 13c,
- de découpes 15 formant les moyens de poussée 14.

En pratique, les découpes 15, 19a et 19b peuvent être réalisées au moyen de tiroirs transversaux.

Lorsque le manchon protecteur 4 tubulaire du connecteur de sécurité 1 à aiguille est fabriqué par injection plastique, celui-ci présente un angle de dépouille lui conférant une paroi latérale périphérique 4a légèrement tronconique.

Lors de son utilisation par un opérateur, par exemple pour la réalisation d'un prélèvement sanguin, le connecteur de sécurité 1 à aiguille est fourni avec ses premier et deuxième volets 12a et 12b en position de protection (figures 6 à 8). Dans cette position, les volets 12a et 12b, en saillie dans l'espace intérieur 7 du manchon protecteur 4 tubulaire au niveau du tronçon distal 11 du manchon protecteur 4 tubulaire, interdisent à un objet ou à un doigt de venir en appui contre l'aiguille 2 en étant introduit dans le manchon protecteur 4 tubulaire par son extrémité distale 8 ouverte. En effet, ledit objet ou le doigt de l'opérateur vient alors en appui selon l'axe longitudinal I-I, contre les premier et deuxième volets 12a et 12b et ne peut pas pénétrer jusque dans le tronçon proximal 10 du manchon protecteur 4 tubulaire.

Lorsque les premier et deuxième volets 12a et 12b sont en position de protection, on voit plus particulièrement sur la figure 8 que la distance D4 séparant les première et deuxième zones de charnière 13a et 13b est inférieure au cumul des longueurs d'arc LC2 et LC1 des premier et deuxième volets 12a et 12b. De ce fait, les premier et deuxième volets 12a et 12b se trouvent dans une position d'équilibre stable.

Lorsque l'opérateur souhaite engager dans l'espace intérieur 7 un tube de prélèvement, il commence par déplacer les premier et deuxième volets 12a et 12b depuis leur position de protection (figures 6 à 8) vers leur position de retrait (figures 1 à 5). Ce déplacement peut être effectué par un doigt de l'opérateur venant directement en appui contre les premier et deuxième volets 12a et 12b et/ou contre la troisième zone de charnière 13c pour déplacer la troisième zone de charnière 13c transversalement selon l'axe transversal VI-VI par un mouvement illustré par la flèche 21 sur la figure 8.

En alternative, l'opérateur peut utiliser les moyens de poussée 14, qui limitent efficacement les risques qu'un doigt de l'opérateur vienne en contact avec l'aiguille 2. En pratique, l'opérateur applique un effort de poussée, illustré par la flèche 22 sur la figure 2 sur la zone de poussée 17 de la languette 16 de façon à déplacer cette zone de poussée 17 transversalement selon l'axe transversal VI-VI en direction de la troisième zone de charnière 13c. Au bout d'une certaine course, la zone de poussée 17 vient au contact de la troisième zone de charnière 13c et/ou des premier et deuxième volets 12a et 12b (figures 9 et 10). En poursuivant la poussée illustrée par la flèche 22 sur la figure 2, l'opérateur repousse les premier et deuxième volets 12a et 12b depuis leur position de saillie vers leur position de retrait (figures 1 à 5).

La position de retrait des premier et deuxième volets 12a et 12b est également une position d'équilibre stable, le cumul des longueurs d'arc LC1 et LC2 des premier et deuxième volets 12a et 12b étant supérieur à la distance D4 séparant les première et deuxième zones de charnière 13a et 13b.

Lors du déplacement des premier et deuxième volets 12a et 12b depuis leur position de protection vers leur position de retrait, les premier et deuxième volets 12a et 12b se déforment élastiquement (en prenant une courbure plus prononcée) et/ou le tronçon distal 11 du manchon protecteur 4 tubulaire se déforme légèrement et élastiquement (en s'ovalisant). Cette ou ces déformations élastiques permettent le passage des premier et deuxième volets 12a et 12b depuis leur position de saillie vers leur position de retrait (et inversement) bien que le cumul des longueurs d'arc LC1 et LC2 soit supérieur à la distance D4 séparant les première et deuxième zones de charnière 13a et 13b. La capacité de déformation des volets 12a et 12b et/ou du tronçon distal 11 de manchon protecteur 4 tubulaire peut être optimisée par le choix d'un matériau plastique adapté (polypropylène copolymère ou polyacétal par exemple), par des épaisseurs de matériau adaptées, ainsi que par une distance appropriée entre les volets 12a et 12b et l'épanouissement radial 18 de rigidification.

Les positions de retrait et de protection sont ainsi des positions d'équilibres stables pour les premier et deuxième volets 12a et 12b. En d'autres termes, lorsque les premier et deuxième volets 12a et 12b sont légèrement déplacés depuis leur position de retrait vers leur position de protection qu'ils occupent (ou inversement depuis leur position protection vers leur position de retrait), ceux-ci ont tendance à revenir dans leur position de retrait (ou de protection). Ce n'est qu'au-delà d'un déplacement selon une amplitude prédéterminée des premier et deuxième volets 12a et 12b, que ceux-ci passent de leur position de retrait à leur position de protection (ou inversement de leur position de protection à leur position de retrait).

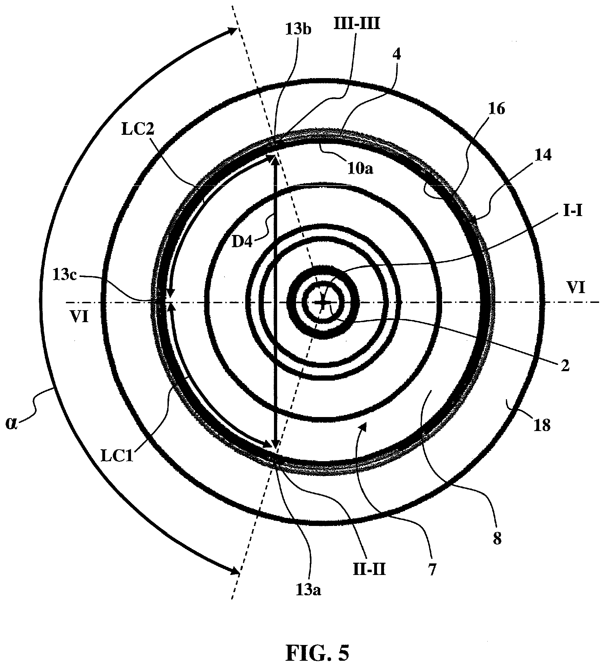

Une fois que les premier et deuxième volets 12a et 12b sont en position de retrait, ceux-ci sont alignés avec la paroi périphérique proximale 10a, de sorte qu'ils ne dépassent plus dans l'espace intérieur 7 du manchon protecteur 4 tubulaire (figure 5). L'espace intérieur 7 est ainsi disponible, et reste disponible du fait de l'équilibre stable des premier et deuxième volets 12a et 12b en position de retrait, pour recevoir un tube de prélèvement enfoncé par l'opérateur dans le manchon protecteur 4 tubulaire à travers son extrémité distale 8 ouverte.

Après le prélèvement, l'opérateur retire le tube de prélèvement hors du manchon protecteur 4 tubulaire et doit jeter le connecteur de sécurité 1 qui est à usage unique. Toutefois, avant de jeter le connecteur de sécurité 1 à aiguille, il est important que l'opérateur s'assure que personne ne puisse venir se piquer sur l'aiguille 2 par inadvertance. Pour ce faire, l'opérateur applique une force de poussée radiale illustrée par la flèche 23 sur la figure 2 sur les premier et deuxième volets 12a et 12b, de façon à déplacer ceux-ci depuis leur position de retrait vers leur position de protection. Une fois les premier et deuxième volets 12a et 12b en position de protection, l'opérateur peut jeter le connecteur de sécurité 1. En l'absence d'une sollicitation volontaire visant à déplacer les premier et deuxième volets 12a et 12b vers leur position de retrait, ceux-ci restent en position de protection du fait de leur équilibre stable en position de protection.

La portion dépassante d'aiguille 6 est enveloppée par une gaine protectrice 20 perforable en élastomère qui, lorsqu'un tube de prélèvement vient se faire perforer par l'aiguille 2, se retrousse le long de l'aiguille 2 en direction de l'extrémité proximale 9 du manchon protecteur 4 tubulaire. La gaine protectrice 20 dépasse dans le tronçon distal 11 mais n'est pas située en correspondance des volets 12a et 12b, pour ne pas gêner les mouvements de ces derniers.

Dans le mode de réalisation illustré sur les figures 1 à 10 la canalisation 5 de conduction de fluide est plutôt destinée à être raccordée à l'extrémité d'un tuyau dont l'autre extrémité est raccordée à une aiguille destinée à pénétrer dans une veine du patient.

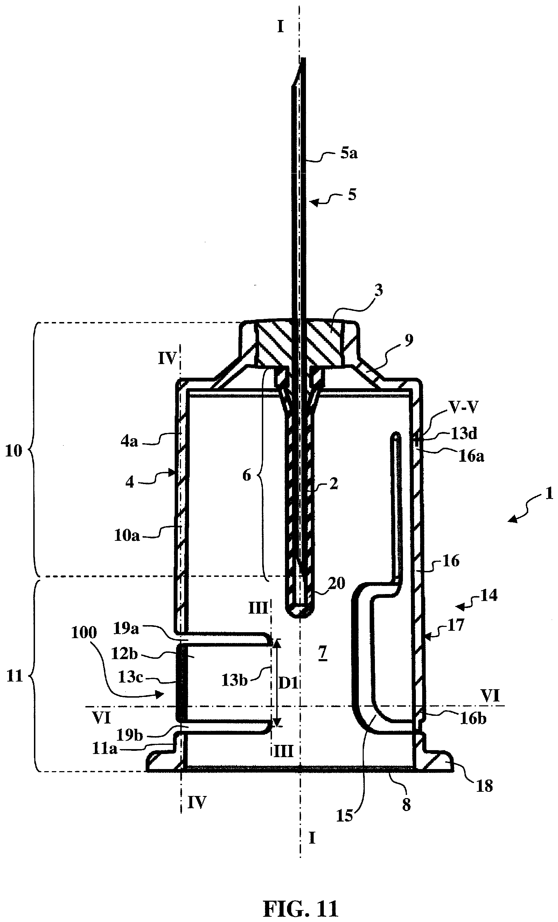

En alternative, comme illustré dans le mode de réalisation particulier de la figure 11, la canalisation 5 de conduction de fluide peut comprendre une portion dépassante d'aiguille 5a similaire à la portion dépassante d'aiguille 6. Sur la figure 11, la portion dépassante d'aiguille 5a ne forme qu'une seule et même aiguille avec la portion dépassante d'aiguille 6. On parle alors d'aiguille 2 double. Le manchon 3 porteur d'aiguille est surmoulé autour de l'aiguille 2 double.

Le connecteur de sécurité 1 peut alors être utilisé avec sa portion dépassante d'aiguille 5a engagée directement dans la veine d'un patient lors d'une prise de sang.

La présente invention n'est pas limitée aux modes de réalisation qui ont été explicitement décrits, mais elle en inclut les diverses variantes et généralisations contenues dans le domaine des revendications ci-après.

## Revendications

1. Connecteur de sécurité (1) à aiguille, comprenant une aiguille (2) creuse de passage de fluide, un manchon (3) support d'aiguille, et un manchon protecteur (4) tubulaire s'étendant selon un axe longitudinal médian (I-I), l'aiguille (2) étant raccordée de manière étanche à une canalisation (5) de conduction de fluide à laquelle est fixé le manchon (3) support d'aiguille, une portion dépassante d'aiguille (6) prolongeant axialement le manchon (3) support d'aiguille à l'opposé de la canalisation (5) de conduction de fluide, le manchon protecteur (4) tubulaire ayant un espace intérieur (7) défini par une paroi périphérique de manchon (4a) et contenant la portion dépassante d'aiguille (6), avec une extrémité distale (8) ouverte pour l'introduction d'un récipient de prélèvement à connecter par l'aiguille (2), et avec une extrémité proximale (9) que traverse le manchon (3) support d'aiguille, le manchon protecteur (4) tubulaire comportant un tronçon proximal (10) à paroi périphérique proximale (10a) et se développant depuis son extrémité proximale (9) en correspondance de la portion dépassante d'aiguille (6), et comportant un tronçon distal (11) à paroi périphérique distale (11a) et prolongeant le tronçon proximal (10) entre la portion dépassante d'aiguille (6) et l'extrémité distale (8) du manchon protecteur (4) tubulaire,
**caractérisé en ce que** :
- la paroi périphérique distale (11a) comporte au moins une portion de paroi sélectivement déplaçable (100) entre une position de retrait et une position de protection,
- en position de retrait, la portion de paroi déplaçable (100) ne dépasse pas dans l'espace intérieur (7),
- en position de protection, la portion de paroi déplaçable (100) fait saillie dans l'espace intérieur (7) pour constituer un obstacle obstruant au moins en partie l'espace intérieur (7) dans le tronçon distal (11).

2. Connecteur de sécurité (1) à aiguille selon la revendication 1, **caractérisé en ce que**, en position de protection, la portion de paroi déplaçable (100) est au moins en partie située au voisinage de l'axe longitudinal médian (I-I), en prolongement de la portion dépassante d'aiguille (6) dans l'espace intérieur (7) du manchon protecteur (4) tubulaire.

3. Connecteur de sécurité (1) à aiguille selon l'une des revendications 1 ou 2, **caractérisé en ce que** :
- la portion de paroi déplaçable (100) comporte des premier (12a) et deuxième (12b) volets ménagés dans la paroi périphérique distale (11a),
- les premier et deuxième volets (12a, 12b) sont respectivement articulés au reste de la paroi périphérique distale (11a) selon une première (13a) et une seconde (13b) zones de charnière respectives autorisant un pivotement des premier (12a) et deuxième (12b) volets autour d'un premier (II-II) et d'un deuxième (III-III) axes de pivotement respectifs parallèles à l'axe longitudinal (I-I),
- les premier (12a) et deuxième (12b) volets sont articulés l'un à l'autre selon une troisième zone de charnière (13c) autorisant un pivotement relatif des premier (12a) et deuxième (12b) volets l'un par rapport à l'autre autour d'un troisième axe de pivotement (IV-IV) parallèle à l'axe longitudinal (I-I),
- les premier (12a) et deuxième (12b) volets sont déplaçables à pivotement, autour des premier (II-II) et deuxième (III-III) axes de pivotement, entre une position de retrait et une position de protection,
- en position de retrait, les premier (12a) et deuxième (12b) volets sont alignés avec la paroi périphérique proximale (10a),
- en position de protection, les premier (12a) et deuxième (12b) volets font saillie dans l'espace intérieur (7) du manchon protecteur (4) tubulaire.

4. Connecteur de sécurité (1) à aiguille selon la revendication 3, **caractérisé en ce que** les première (13a), deuxième (13b) et troisième (13c) zones de charnière sont réalisées au moyen d'un amincissement local de l'épaisseur de matériau de la paroi périphérique de manchon (4a).

5. Connecteur de sécurité (1) à aiguille selon l'une des revendications 3 ou 4, **caractérisé en ce que** :
- le manchon protecteur (4) tubulaire a une section transversale circulaire,
- les premier (II-II) et deuxième (III-III) axes de pivotement sont séparés l'un de l'autre par un secteur angulaire inférieur à 180°.

6. Connecteur de sécurité (1) à aiguille selon la revendication 5, **caractérisé en ce que** les premier (II-II) et deuxième (III-III) axes de pivotement sont séparés l'un de l'autre par un secteur angulaire (a) compris entre environ 120° et environ 170°.

7. Connecteur de sécurité (1) à aiguille selon l'une quelconque des revendications 1 à 6, **caractérisé en ce que** la portion de paroi déplaçable (100) s'étend, le long de l'axe longitudinal (I-I), selon une distance (D1) comprise entre 12 mm environ et 15 mm environ.

8. Connecteur de sécurité (1) à aiguille selon l'une quelconque des revendications 1 à 7, **caractérisé en ce que** :
- le connecteur de sécurité (1) à aiguille comporte des moyens de poussée (14), ménagés dans la paroi périphérique distale (11a) et/ou dans la paroi périphérique proximale (10a), et situés au moins en partie en correspondance de la portion de paroi déplaçable (100),
- les moyens de poussée (14) sont au moins en partie déplaçables transversalement entre une position de repos et au moins une position de poussée, en étant rappelés élastiquement vers leur position de repos,
- en position de repos, les moyens de poussée (14) ne dépassent pas dans l'espace intérieur (7) et sont de préférence alignés avec la paroi périphérique proximale (10a),
- en position de poussée, les moyens de poussée (14) font saillie dans l'espace intérieur (7) du manchon protecteur (4) tubulaire pour solliciter la portion de paroi déplaçable (100) et la rappeler en position de retrait.

9. Connecteur de sécurité (1) à aiguille selon la revendication 8, **caractérisé en ce que** :
- les moyens de poussée (14) se présentent sous la forme d'une languette (16) s'étendant selon l'axe longitudinal (I-I) entre une première extrémité (16a) et une deuxième extrémité (16b) libre,
- la languette (16) est articulée à sa première extrémité (16a) sur le reste de la paroi périphérique proximale (10a) et/ou de la paroi périphérique distale (11a) selon une quatrième zone de charnière (13d) autorisant un pivotement de la languette (16) autour d'un quatrième axe de pivotement (V-V) perpendiculaire à l'axe longitudinal (I-I),
- la deuxième extrémité (16b) libre de la languette (16) est située en correspondance de la portion de paroi déplaçable (100).

10. Connecteur de sécurité (1) à aiguille selon la revendication 9, **caractérisé en ce que** la deuxième extrémité (16b) libre de la languette (16) comporte une zone de poussée (17) qui s'étend, le long de l'axe longitudinal (I-I), selon une distance (D2) comprise entre 12 mm environ et 15 mm environ, et qui s'étend, perpendiculairement à l'axe longitudinal (I-I), selon une distance (D3) comprise entre 10 mm environ et 18 mm environ.

11. Connecteur de sécurité (1) à aiguille selon l'une quelconque des revendications 8 à 10, **caractérisé en ce que** les moyens de poussée (14) sont situés de façon diamétralement opposée à la portion de paroi déplaçable (100) par rapport à l'axe longitudinal (I-I).

12. Connecteur de sécurité (1) à aiguille selon l'une quelconque des revendications 1 à 11, **caractérisé en ce que** le manchon protecteur (4) tubulaire est en matière plastique.

13. Connecteur de sécurité (1) à aiguille selon l'une quelconque des revendications 1 à 12, **caractérisé en ce que** :
- le manchon protecteur (4) tubulaire comporte, à son extrémité distale (8) ouverte, un épanouissement radial (18),
- la portion de paroi déplaçable (100) est ménagée, selon l'axe longitudinal (I-I), à proximité immédiate de l'extrémité distale (8) ouverte.

14. Connecteur de sécurité (1) à aiguille selon l'une quelconque des revendications 1 à 13, **caractérisé en ce que** le manchon protecteur (4) tubulaire est surmoulé sur le manchon (3) support d'aiguille.

15. Procédé de fabrication d'un connecteur de sécurité (1) à aiguille selon l'une quelconque des revendications 1 à 14, **caractérisé en ce qu'**il comporte une étape d'injection de matière plastique au cours de laquelle on forme, en une seule fois, le manchon protecteur (4) tubulaire muni :
- de découpes (19a, 19b), et le cas échéant d'amincissements d'épaisseur de matière, formant la portion de paroi déplaçable (100),
- le cas échéant, de découpes (15) formant les moyens de poussée (14).

## Patentansprüche

1. Sicherheitsverbinder (1) mit Nadeln, umfassend eine hohle Nadel (2) zum Durchlassen eines Fluides, eine Nadelstützhülse (3), und eine rohrförmige Schutzhülse (4), die sich entlang einer zentralen Längsachse (I-I) erstreckt, wobei die Nadel (2) in einer leckagedichten Weise mit einem fluidtragenden Kanal (5) verbunden ist, an dem die Nadelstützhülse (3) befestigt ist, einen vorspringenden Nadelbereich (6) der axial die Nadelstützhülse (3) an einem Ende gegenüberliegend dem fluidtragendem Kanal (5) weiterführt, wobei die röhrenförmige Schutzhülse (4) einen Innenraum (7), welcher durch eine äußere Hülsenwand (4a) definiert ist und den vorspringenden Nadelbereich (6) enthält, mit einem offenen distalen Ende (8) zum Einführen eines durch die Nadel (2) zu verbindenden Probenbehälters und mit einem proximalen Ende (9) durch den die Nadelstützhülse (3) tritt umfasst, wobei die röhrenförmige Schutzhülse (4) einen proximalen Abschnitt (10) mit einer proximalen äußeren Wand (10a) umfasst, die sich von ihrem proximalen Ende (9) zusammen mit dem vorspringenden Nadelbereich (6) herausbildet, und die einen distalen Abschnitt (11) mit einer distalen äußeren Wand (11a) umfasst, die den proximalen Abschnitt (10) zwischen dem vorspringendem Nadelbereich (6) und dem distalen Ende (8) der röhrenförmigen Schutzhülse (4) weiterführt,
**dadurch gekennzeichnet, dass**:
- die distale äußere Wand (11a) wenigstens einen Wandbereich (100) aufweist, der wahlweise zwischen einer eingezogenen Position und einer Schutzposition beweglich ist,
- in der eingezogenen Position der bewegliche Wandbereich (100) noch in den Innenraum (7) vorspringt,
- in der Schutzposition der bewegliche Wandbereich (100) in den Innenraum (7) vorspringt, um ein Hindernis zu bilden, das wenigstens teilweise den Innenraum (7) in dem distalen Abschnitt (11) blockiert.

2. Sicherheitsverbinder (1) mit Nadeln nach Anspruch 1, **dadurch gekennzeichnet, dass** in der Schutzposition sich der bewegliche Wandbereich (100) wenigstens teilweise in der Nähe der zentralen Längsachse (I-I) befindet, in einer Fortführung des vorspringenden Nadelbereiches (6) im Innenraum (7) der röhrenförmigen Schutzhülse (4).

3. Sicherheitsverbinder (1) mit Nadeln nach einem der Anspruche 1 oder 2, **dadurch gekennzeichnet, dass**:
- der bewegliche Wandabschnitt (100) eine erste (12a) und eine zweite (12b) Klappe aufweist, die an der distalen äußeren Wand (11a) ausgebildet sind,
- die erste und zweite Klappe (12a, 12b) entsprechend an den Rest der distalen äußeren Wand (11a) in einer ersten Gelenkzone (13a) und einer zweiten Gelenkzone (13b) angebunden sind, was ein Schwenken der ersten (12a) und zweiten (12b) Klappe entsprechend um eine erste Schwenkachse (II-II) und eine zweite Schwenkachse (III-III) parallel zur Längsachse (I-I) erlaubt,
- die erste (12a) und zweite (12b) Klappe aneinander in einer dritten Gelenkzone (13c) angebunden sind, was ein relatives Verschwenken der ersten (12a) und der zweiten (12b) Klappe gegeneinander um eine dritte Schwenkachse (IV-IV) parallel zur Längsachse (I-I) erlaubt,
- wobei die erste (12a) und zweite (12b) Klappe durch Verschwenken um die erste (II-II) und zweite (III-III) Schwenkachse zwischen einer eingezogenen Position und einer Schutzposition beweglich sind,
- wobei in der in der eingezogenen Position die erste (12a) und zweite (12b) Klappe an der proximalen äußeren Wand (10a) ausgerichtet sind,
- wobei in der Schutzposition die erste (12a) und zweite (12b) Klappe in den Innenraum (7) der röhrenförmigen Schutzhülse (4) ragt.

4. Sicherheitsverbinder (1) mit Nadeln nach Anspruch 3, **dadurch gekennzeichnet, dass** die erste (13a), zweite (13b) und dritte (13c) Gelenkzone durch eine lokale Verjüngung der Materialstärke der periphären Hülsenwand (4a) gefertigt sind.

5. Sicherheitsverbinder (1) mit Nadeln nach einem der Ansprüche 3 oder 4, **dadurch gekennzeichnet, dass**:
- die röhrenförmige Schutzhülse (4) einen kreisförmigen Querschnitt aufweist,
- die erste (II-II) und zweite (III-III) Schwenkachse voneinander durch einen Winkelsektor von weniger als 180° getrennt sind.

6. Sicherheitsverbinder (1) mit Nadeln nach Anspruch 5, **dadurch gekennzeichnet, dass** die erste (II-II) und zweite (III-III) Schwenkachse voneinander durch einen Winkelsektor (α) zischen ungefähr 120° und ungefähr 170° getrennt sind.

7. Sicherheitsverbinder (1) mit Nadeln nach einem der Ansprüche 1 bis 6, **dadurch gekennzeichnet, dass** sich der bewegliche Wandabschnitt (100) entlang der Längsachse (I-I) über eine Strecke (D1) zwischen ungefähr 12 mm und ungefähr 15 mm erstreckt.

8. Sicherheitsverbinder (1) mit Nadeln nach einem der Ansprüche 1 bis 7, **dadurch gekennzeichnet, dass**
- der Sicherheitsverbinder (1) mit Nadeln Stoßmittel (14) aufweist, die in der distalen äußeren Wand (11a) und/oder in der proximalen äußeren Wand (10a) ausgebildet sind, und die wenigstens teilweise auf mit dem beweglichen Wandabschnitt (100) ausgerichtet sind,
- die Stoßmittel (14) wenigstens teilweise quer zwischen einer Ruheposition und wenigstens einer Stoßposition beweglich sind, aus der sie elastisch in ihre Ruheposition zurückgesetzt werden,
- in der Ruheposition die Stoßmittel (14) nicht in den Innenraum (7) ragen und vorzugsweise mit der proximalen äußeren Wand (10a) ausgerichtet sind,
- in der Stoßposition die Stoßmittel (14) in den Innenraum (7) der röhrenförmigen Schutzhülse (4) ragen, um dden beweglichen Wandabschnitt (100) zu belasten und ihn in die eingezogene Position zurückzusetzen.

9. Sicherheitsverbinder (1) mit Nadeln nach Anspruch 8, **dadurch gekennzeichnet, dass**:
- die Stoßmittel (14) als Zunge (16) ausgebildet sind, die sich entlang der Längsachse (I-I) zwischen einem ersten Ende (16a) und einem freien zweiten Ende (16b) erstreckt,
- die Zunge (16) an ihrem ersten Ende (16a) an den Rest der proximalen äußeren Wand (10a) und/oder der distalen äußeren Wand (11a) in einer vierten Gelenkzone (13d) angebunden sind, was ein Schwenken der Zunge (16) um eine vierte Schwenkachse (V-V) rechtwinklig zur Längsachse (I-I) ermöglicht,
- das freie zweite Ende (16b) der Zunge (16) sich ausgerichtet zum beweglichen Wandabschnitt (100) befindet.

10. Sicherheitsverbinder (1) mit Nadeln nach Anspruch 9, **dadurch gekennzeichnet, dass** das freie Ende (16b) der Zunge (16) eine Stoßzone (17) aufweist, die sich entlang der Längsachse (I-I) über eine Strecke (D2) zwischen ungefähr 12 mm und ungefähr 15 mm erstreckt und sich rechtwinklig zur Längsachse (I-I) über eine Strecke (D3) zwischen ungefähr 10 mm und ungefähr 18 mm erstreckt.

11. Sicherheitsverbinder (1) mit Nadeln nach einem der Ansprüche 8 bis 10, **dadurch gekennzeichnet, dass** sich die Stoßmittel (14) entgegengesetzt verlaufend zum beweglichen Wandabschnitt (100) in Bezug zur Längsachse (I-I) befinden.

12. Sicherheitsverbinder (1) mit Nadeln nach einem der Ansprüche 1 bis 11, **dadurch gekennzeichnet, dass** die röhrenförmige Schutzhülse (4) aus Plastik gefertigt ist.

13. Sicherheitsverbinder (1) mit Nadeln nach einem der Ansprüche 1 bis 12, **dadurch gekennzeichnet, dass**:
- die röhrenförmige Schutzhülse (4) an ihrem offenen distalen Ende (8) eine radiale Aufweitung (18) aufweist,
- der bewegliche Wandabschnitt (100) entlang der Längsachse (I-I) in unmittelbarer Nähe zum offenen distalen Ende (8) ausgebildet ist.

14. Sicherheitsverbinder (1) mit Nadeln nach eienm der Ansprüche 1 bis 13, **dadurch gekennzeichnet, dass** die röhrenförmige Schutzhülse (4) auf die Nadelstützhülse (3) aufgegossen ist.

15. Verfahren zum Herstellen eines Sicherheitsverbinders (1) mit Nadeln nach einem der Ansprüche 1 bis 14, **dadurch gekennzeichnet, dass** es einen Plastikeinspritzschritt umfasst, in dem die röhrenförmige Schutzhülse (4) in einem Schritt gebildet und ausgestattet wird mit:
- Stanzteile (19a, 19b), und falls geeignet mit einer einer reduzierten Materialstärke, die den beweglichen Wandabschnitt (100) bilden,
- falls geeignet, Stanzteile (15), die die Stoßmittel (14) bilden.

## Claims

1. A safety connector (1) with needle, comprising a hollow needle (2) for passage of fluid, a needle support sleeve (3), and a tubular protective sleeve (4) extending along a central longitudinal axis (I-I), the needle (2) being connected in a leaktight manner to a fluid-carrying channel (5) to which the needle support sleeve (3) is fixed, a protruding needle portion (6) axially continuing the needle support sleeve (3) at the end opposite the fluid-carrying channel (5), the tubular protective sleeve (4) having an inner space (7) defined by a peripheral sleeve wall (4a) and containing the protruding needle portion (6), with an open distal end (8) for the introduction of a sampling container to be connected by the needle (2), and with a proximal end (9) through which the needle support sleeve -(3) passes, the tubular protective sleeve (4) having a proximal segment (10) with a proximal peripheral wall (10a) developing from its proximal end (9) in line with the protruding needle portion (6), and having a distal segment (11) with a distal peripheral wall (11a) continuing the proximal segment (10) between the protruding needle portion (6) and the distal end (8) of the tubular protective sleeve (4),
**characterized in that**:
- the distal peripheral wall (11a) has at least one wall portion (100) selectively movable between a retracted position and a protection position,
- in the retracted position, the movable wall portion (100) does not protrude into the inner space (7),
- in the protection position, the movable wall portion (100) projects into the inner space (7) in order to form an obstacle that at least partially blocks the inner space (7) in the distal segment (11).

2. The safety connector (1) with needle as claimed in claim 1, **characterized in that**, in the protection position, the movable wall portion (100) is at least partially situated in the vicinity of the central longitudinal axis (I-I), in a continuation of the protruding needle portion (6) in the inner space (7) of the tubular protective sleeve (4).

3. The safety connector (1) with needle as claimed in either of claims 1 and 2, **characterized in that**:
- the movable wall portion (100) has first (12a) and second (12b) flaps formed in the distal peripheral wall (11a),
- the first and second flaps (12a, 12b) are respectively articulated on the rest of the distal peripheral wall (11a) in a first hinge zone (13a) and a second hinge zone (13b), respectively permitting a pivoting of the first (12a) and second (12b) flaps about a first pivot axis (II-II) and a second pivot axis (III-III) parallel to the longitudinal axis (I-I),
- the first (12a) and second (12b) flaps are articulated on each other in a third hinge zone (13c), permitting a relative pivoting of the first (12a) and second (12b) flaps with respect to each other about a third pivot axis (IV-IV) parallel to the longitudinal axis (I-I),
- the first (12a) and second (12b) flaps are movable by pivoting, about first (II-II) and second (III-III) pivot axes, between a retracted position and a protection position,
- in the retracted position, the first (12a) and second (12b) flaps are aligned with the proximal peripheral wall (10a),
- in the protection position, the first (12a) and second (12b) flaps project into the inner space (7) of the tubular protective sleeve (4).

4. The safety connector (1) with needle as claimed in claim 3, **characterized in that** the first (13a), second (13b) and third (13c) hinge zones are produced by a local thinning of the material thickness of the peripheral sleeve wall (4a).

5. The safety connector (1) with needle as claimed in either of claims 3 and 4, **characterized in that**:
- the tubular protective sleeve (4) has a circular cross section,
- the first (II-II) and second (III-III) pivot axes are separated from each other by an angle sector of less than 180°.

6. The safety connector (1) with needle as claimed in claim 5, **characterized in that** the first (II-II) and second (III-III) pivot axes are separated from each other by an angle sector (α) of between about 120° and about 170°.

7. The safety connector (1) with needle as claimed in any one of claims 1 through 6, **characterized in that** the movable wall portion (100) extends, along the longitudinal axis (I-I), by a distance (D1) of between about 12 mm and about 15 mm.

8. The safety connector (1) with needle as claimed in any one of claims 1 through 7, **characterized in that**:
- the safety connector (1) with needle has pushing means (14) which are formed in the distal peripheral wall (11a) and/or in the proximal peripheral wall (10a) and are situated at least partially in line with the movable wall portion (100),
- the pushing means (14) are at least partially movable transversely between a rest position and at least one pushing position, being returned elastically to their rest position,
- in the rest position, the pushing means (14) do not protrude into the inner space (7) and are preferably aligned with the proximal peripheral wall (10a),
- in the pushing position, the pushing means (14) project into the inner space (7) of the tubular protective sleeve (4) in order to stress the movable wall portion (100) and return it to the retracted position.

9. The safety connector (1) with needle as claimed in claim 8, **characterized in that**:
- the pushing means (14) are in the form of a tongue (16) extending along the longitudinal axis (I-I) between a first end (16a) and a free second end (16b),
- the tongue (16) is articulated at its first end (16a) on the rest of the proximal peripheral wall (10a) and/or of the distal peripheral wall (11a) in a fourth hinge zone (13d), permitting a pivoting of the tongue (16) about a fourth pivot axis (V-V) perpendicular to the longitudinal axis (I-I),
- the free second end (16b) of the tongue (16) is situated in line with the movable wall portion (100).

10. The safety connector (1) with needle as claimed in claim 9, **characterized in that** the free second end (16b) of the tongue (16) has a pushing zone (17) which extends, along the longitudinal axis (I-I), by a distance (D2) of between about 12 mm and about 15 mm and extends, perpendicularly with respect to the longitudinal axis (I-I), by a distance (D3) of between about 10 mm and about 18 mm.

11. The safety connector (1) with needle as claimed in any one of claims 8 through 10, **characterized in that** the pushing means (14) are situated diametrically opposite the movable wall portion (100) in relation to the longitudinal axis (I-I) .

12. The safety connector (1) with needle as claimed in any one of claims 1 through 11, **characterized in that** the tubular protective sleeve (4) is made of plastic.

13. The safety connector (1) with needle as claimed in any one of claims 1 through 12, **characterized in that**:
- the tubular protective sleeve (4) has, at its open distal end (8), a radial widening (18),
- the movable wall portion (100) is formed, along the longitudinal axis (I-I), in immediate proximity to the open distal end (8).

14. The safety connector (1) with needle as claimed in any one of claims 1 through 13, **characterized in that** the tubular protective sleeve (4) is overmolded on the needle support sleeve (3).

15. A method for producing a safety connector (1) with needle as claimed in any one of claims 1 through 14, **characterized in that** it comprises a step of plastic injection molding, during which the tubular protective sleeve (4) is formed in one go and provided with:
- cutouts (19a, 19b), and if appropriate a reduced material thickness, forming the movable wall portion (100),
- if appropriate, cutouts (15) forming the pushing means (14).
